# EUROPÄISCHE PATENTANMELDUNG

(11) **EP 1 248 105 A2**
(43) Veröffentlichungstag der Anmeldung: **09.10.2002**
(21) Anmeldenummer: 02005694.1
(22) Anmeldetag: 13.03.2002
(51) Int. Cl.: G01N 33/46

(54) **Verfahren und Vorrichtung zum Messen der Feuchte in einer Anzahl von Werkstücken**

(30) Priorität: 16.03.2001 DE 10113705
(71) Anmelder: Calgaro, Alberto, 71732 Tamm (DE)
(72) Erfinder: Calgaro, Alberto, 71732 Tamm (DE)
(74) Vertreter: Hössle & Kudlek

(57) **Zusammenfassung**

Es wird ein Verfahren zum Messen der Feuchte in eine Anzahl von Werkstücken (13) vorgestellt, bei dem die Werkstücke (13) nacheinander an einem Sensor (16) vorbeigeführt werden und in jeweils einem Meßzyklus die Feuchte in einem der Werkstücke (13) mindestens einmal gemessen wird, während das betreffende Werkstück (13) an dem Sensor (16) vorbeigeführt wird, wobei die Feuchte mittels eines mit dem Sensor (16) verbundenen Meßsystems (11) ermittelt wird. Das erfindungsgemäße Verfahren ist dadurch gekennzeichnet, daß nach jedem Meßzyklus ein Abgleich des Meßsystems (11) durchgeführt wird. Das erfindungsgemäße Verfahren ist insbesondere zum Messen der Feuchte in Holz geeignet.

## Beschreibung

Die Erfindung betrifft ein Verfahren zum Messen der Feuchte in einer Anzahl von Werkstücken nach dem Oberbegriff des Anspruchs 1, eine Vorrichtung zur Durchführung dieses Verfahrens nach dem Oberbegriff des Anspruchs 8 und ein Meßsystem zur Verwendung in der erfindungsgemäßen Vorrichtung.

Die Feuchte eines Werkstücks gibt die in einer Volumeneinheit des Werkstücks enthaltene Wasserdampfmenge wieder. Da die Feuchte ein Faktor ist, der die Eigenschaften des Werkstücks bestimmt, ist es wichtig, die Feuchte bzw. den Feuchtegehalt zu kennen. Insbesondere bei Naturprodukten wie Holz ist eine Bestimmung bzw. Messung der Feuchte unerläßlich.

Holz ist ein beliebter Werkstoff, der in vielen Bereichen eingesetzt wird. Bei der Produktion von Holz als Werk- bzw. Baustoff ist es erforderlich, in regelmäßigen Abständen Qualitätskontrollen durchzuführen, um gewünschte Eigenschaften des Endprodukts sicherzustellen. So muß die Feuchte in einem von der Anwendung abhängigen Bereich liegen. Aus diesem Grunde wird bei der Holzproduktion und Holzverarbeitung die Feuchte des zu verarbeitenden Holzes gemessen.

Zur Bestimmung der Feuchte in Holz werden Meßanlagen zum Messen der Feuchte in Holzbrettern, Holzbalken und Furnieren eingesetzt. Hierbei ist erforderlich, diese Werkstücke in großer Anzahl in möglichst kurzer Zeit zu untersuchen. Bekannte Meßanlagen bieten zudem die Möglichkeit, nach dem Ermitteln der Feuchtigkeit das Meßgut auf Wunsch zu markieren und/oder auszusortieren. Dies geschieht durch den Vergleich zwischen dem Ist-Wert des Meßguts und den Sortiergrenzen (Soll-Wert). Herkömmliche Meßanlagen können auch in schon bestehende Förderbandsysteme des Unternehmens mit einbezogen werden.

Da es wichtig ist, nicht nur den Mittelwert der Feuchte in einem Holzbrett zu bestimmen, sondern es vielmehr erforderlich ist, ein Feuchteprofil des jeweiligen Holzbretts zu erstellen, messen bekannte Meßanlagen die Feuchte in einem Holzbrett an verschiedenen Stellen. Es ist üblich, Holzbretter an einem Sensor vorbeizuführen, der in regelmäßigen Abständen einen die Feuchte repräsentierenden Wert in dem Bereich bestimmt, der zum Meßzeitpunkt von dem Sensor erfaßt wird.

Man unterscheidet zwischen kontaktlosen Meßverfahren und solchen, bei denen der eingesetzte Sensor das zu messende Holzbrett berührt. Bei den letztgenannten ist von Nachteil, daß sich eventuell an der entsprechenden Stelle auf der Oberfläche des Holzbretts befindliche Wassertropfen die Messung erheblich beeinflussen. Das erhaltene Meßergebnis gibt in einem solchen Fall nicht die tatsächliche Feuchte in dem Werkstück wieder.

Demgegenüber ist bei kontaktlosen Meßverfahren nachteilig, daß sich ändernde Rahmenbedingungen, wie beispielsweise die Luftfeuchtigkeit, die Meßergebnisse verfälschen können. Auch in dem Luftspalt zwischen Holzbrett und Sensor befindliche Sägespäne können das Ergebnis der Messung beeinflussen. Daher ist es bei bekannten Meßanlagen unumgänglich, diese mehrmals am Tag zu kalibrieren, um geänderte Rahmenbedingungen berücksichtigen zu können. Diese Vorgehensweise erweist sich als aufwendig, da bei jeder Kalibrierung der Meßprozeß und damit möglicherweise der Fertigungsprozeß unterbrochen werden muß. Auch sind mit einer solchen Vorgehensweise keine zufriedenstellenden Ergebnisse zu erzielen, da die Meßungenauigkeit nach einer Kalibrierung in Abhängigkeit von der Zeit stetig zunimmt.

Demgegenüber wird erfindungsgemäß ein Verfahren mit den Merkmalen des Anspruchs 1 und eine Vorrichtung zur Durchführung des erfindungsgemäßen Verfahrens mit den Merkmalen des Anspruchs 8 sowie ein Meßsystem zur Verwendung in einer erfindungsgemäßen Vorrichtung mit den Merkmalen des Anspruchs 17 vorgeschlagen. Vorteilhafte Weiterbildungen ergeben sich aus den Unteransprüchen.

Das erfindungsgemäße Verfahren zum Messen der Feuchte in einer Anzahl von Werkstücken, bei dem die Werkstücke nacheinander an einem Sensor vorbeigeführt werden und in jeweils einem Meßzyklus die Feuchte in einem der Werkstücke mindestens einmal gemessen wird, während das Werkstück an dem Sensor vorbeigeführt wird, wobei die Feuchte mittels eines mit dem Sensor verbundenen Meßsystems ermittelt wird, zeichnet sich dadurch aus, daß nach jedem Meßzyklus ein Abgleich des Meßsystems durchgeführt wird.

Das erfindungsgemäße Verfahren ist für beliebige Werkstükke, insbesondere aber für Holzbretter, Holzbalken und Furniere geeignet.

Aufgrund der Tatsache, daß nach jedem Meßzyklus, also immer dann, wenn gerade kein Werkstück zum Messen der Feuchte am Sensor vorbeigeführt wird, ein Abgleich des Meßsystems erfolgt, können bei der Messung geänderte Rahmenbedingungen berücksichtigt werden. Zweckmäßigerweise erfolgt eine Kalibrierung des Meßsystems, bspw. mit einem Handmeßgerät, einmal am Tag zu Beginn der Messungen. Außerdem erfolgt eine Messung ohne Werkstück. Dabei wird ein Wert, der sogenannte Referenzwert, ermittelt. Nach jedem Meßzyklus wird das Meßsystem wieder so eingestellt, daß dieser Referenzwert ermittelt wird. Ein mehrmaliges Kalibrieren des Meßsystems, um auf geänderte Rahmenbedingungen, wie bspw. Luftfeuchtigkeit und Raumtemperatur, zu reagieren, ist nicht erforderlich. Dies spart Zeit und verringert in erheblichem Umfang die Kosten.

Bevorzugt wird die Feuchte mittels eines kapazitiven Meßverfahrens ermittelt. Die Permittivitätszahl von Wasser ist mit εᵣ = 81 sowohl gegenüber der von trockener Luft (εᵣ = 1) als auch gegenüber der Permittivitätszahlen der meisten Isolierstoffe sehr hoch. Eine Einlagerung von Feuchtigkeit in dem Werkstück verändert daher relativ stark die Permittivität und mit ihr die Kapazität. Aus dieser wird dann auf die Feuchtigkeit bzw. Feuchte geschlossen.

Bei einer kapazitiven Messung kann der Sensor eine Elektrode eines Kondensators bilden. Die zugehörige zweite Elektrode ist unter oder im Bereich des zu untersuchenden Werkstücks angeordnet, so daß das Werkstück sich während der Messung im wesentlichen zwischen den beiden Elektroden befindet. Die Kapazität des Kondensators ist maßgeblich von dem zwischen den Elektroden befindlichen Werkstück abhängig. Folglich beeinflußt auch die Feuchte des Werkstücks die ermittelte Kapazität. Da auch die Ausmaße des Werkstücks die Kapazität beeinflussen, ist darauf zu achten, daß sämtliche zu untersuchenden Werkstücke die gleichen Ausmaße haben. Das Meßsystem muß einmal zu Beginn kalibriert werden, um eine Beziehung zwischen ermittelter Kapazität und Feuchte herzustellen. Ist dies erfolgt, kann die Feuchte einer Vielzahl von Werkstücken indirekt durch eine Bestimmung der Kapazität gemessen werden. Durch den Abgleich des Meßsystems nach jedem Meßzyklus werden geänderte Rahmenbedingungen berücksichtigt.

Von Vorteil ist, wenn die Messung kontaktlos erfolgt. Je weiter der Sensor vom Werkstück entfernt ist, desto unabhängiger ist die Messung von Störgrößen.

Um ein Feuchteprofil des Werkstücks erstellen zu können, werden in einem Meßzyklus mehrere Messungen durchgeführt. Die Messungen erfolgen dann in regelmäßigen Abständen. So werden für ein Werkstück mehrere Feuchtewerte ermittelt, die einem bestimmten Bereich des Werkstücks zugeordnet werden können. Mit den ermittelten Werten kann selbstverständlich eine Mittelwertbildung durchgeführt werden.

Es ist möglich, die Werkstücke mit einer Geschwindigkeit von 400 m/min an dem Sensor vorbeizuführen. Die Feuchte jedes Werkstücks kann dann in Abständen von 1/10 mm bestimmt werden. Das bedeutet, daß die Feuchte im Werkstück an unterschiedlichen Stellen gemessen wird und diese Stellen sich im Abstand von 1/10 mm voneinander befinden. Auf diese Weise ist ein genaues Feuchteprofil des Werkstücks zu erstellen.

Die erfindungsgemäße Vorrichtung zur Durchführung des vorstehend beschriebenen Verfahrens weist einen Sensor, ein mit dem Sensor verbundenes Meßsystem und ein Fördermittel zum Vorbeiführen der Werkstücke an dem Sensor auf. Die Vorrichtung zeichnet sich dadurch aus, daß ein Abgleicher zum Abgleichen des Meßsystems vorgesehen ist.

Der Abgleich des Meßsystems erfolgt dabei nach jedem Meßzyklus, das heißt, nach dem Bestimmen der Feuchte eines Werkstücks, wobei jedes Werkstück einmal oder auch mehrmals, also an mehreren Stellen, gemessen werden kann. Zum Abgleichen erfolgt eine sogenannte "Leermessung", d.h. eine Messung ohne Werkstück. Da bekannt ist, welcher Wert zu Beginn der Messung im Rahmen der Kalibrierung ohne Werkstück vom Meßsystem ermittelt wurde, kann durch Einstellen des bekannten Werts das gesamte Meßsystem auf geänderte Rahmenbedingungen abgeglichen werden. Das Meßsystem wird bei jedem Abgleich somit derart eingestellt, daß jede Leermessung denselben Wert, nämlich den Referenzwert, ergibt.

Als Abgleicher eignet sich vorzugsweise ein Mikroprozessor, der mit geringem Zeitaufwand die Einstellungen des Meßsystems ermitteln und präzise einstellen kann.

In einer bevorzugten Ausführungsform weist das Meßsystem eine Hochfrequenzspule auf, an die ein hochfrequentes Eingangssignal anzulegen ist und mit deren einem Anschluß der Sensor und mit deren anderem Anschluß eine Kapazität verbunden ist. Weiterhin ist ein parallel zur Hochfrequenzspule geschaltetes Potentiometer mit einem einstellbaren Abgriff sowie ein Gleichrichter vorgesehen. Der Eingang des Gleichrichters ist mit dem Abgriff des Potentiometers verbunden. Der Ausgang des Gleichrichters gibt das Ausgangssignal des Meßsystems aus. Während der Messung wird ein hochfrequentes Signal an die Hochfrequenzspule angelegt. In Abhängigkeit von dem am Sensor eingestellten Kapazitätswert im Vergleich zum Wert der mit dem anderen Anschluß der Hochfrequenzspule verbundenen Kapazität stellt sich am Abgriff des Potentiometers eine Wechselgröße ein, die mittels des Gleichrichters in eine Gleichgröße, nämlich das Ausgangssignal des Meßsystems, gewandelt wird. Dieses Ausgangssignal ist abhängig von der vom Sensor ermittelten Kapazität des Werkstücks und damit abhängig von dessen Feuchte.

Zu Beginn der Messung wird das System kalibriert. Dabei wird bei einer Leermessung der Abgriff des Potentiometers so eingestellt, daß sich ein möglichst kleines Ausgangssignal, das Referenzsignal, ergibt. Ein kleines Referenzsignal gewährleistet einen großen Dynamikbereich. Dieser Referenzwert dient zum Abgleichen des Meßsystems. Nach jedem Meßzyklus wird das Meßsystem so eingestellt, daß die Leermessung wieder den Referenzwert ergibt. Nach der Festlegung des Referenzwerts kann das Meßsystem, bspw. mit Hilfe eines Handmeßgeräts, kalibriert werden, d.h. es wird eine Beziehung zwischen Wert des Ausgangssignals und Feuchte im Werkstück hergestellt.

Zum Abgleichen des Meßsystems wird nach jedem Meßzyklus das Potentiometer so eingestellt, daß am Ausgang des Gleichrichters das Referenzsignal anliegt.

Versuche haben ergeben, daß das hochfrequente Eingangssignal eine Frequenz von bis zu 50 MHz aufweisen kann.. Je höher die Frequenz ist, desto weiter kann der Sensor vom zu untersuchenden Werkstück entfernt angeordnet sein und desto weniger störanfällig ist die Messung. Insbesondere die Möglichkeit, mit hohen Frequenzen messen zu können, die aus dem Stand der Technik nicht bekannt ist, stellt einen wichtigen Vorteil der Erfindung dar.

Vorteilhafterweise erfolgt eine nochmalige Verstärkung des Ausgangssignals, um eine höhere Genauigkeit zu erzielen. Zweckmäßigerweise ist der Verstärker ebenfalls abgleichbar. Der Verstärker weist in einer bevorzugten Ausführungsform wenigstens einen Operationsverstärker auf.

In bevorzugter Ausgestaltung dient als Fördermittel ein Förderband. Die erfindungsgemäße Vorrichtung kann dann in ein schon bestehendes Förderbandsystem einbezogen werden.

In einer besonders vorteilhaften Ausgestaltung der Erfindung sind zwei Lichtschranken zum Starten und Beenden der Meßzyklen vorgesehen. Die beiden Lichtschranken sind in der Nähe des Sensors angeordnet, und zwar eine vor und eine hinter dem Sensor bezogen auf die Bewegungsrichtung der Werkstücke.

Eine dritte Lichtschranke kann zum Starten des Abgleichvorgangs vorgesehen sein. Diese ist zweckmäßiger weise vor den beiden Lichtschranken bezogen auf die Bewegungsrichtung der Werkstücke angeordnet.

Ein erfindungsgemäßes Meßsystem weist eine Hochfrequenzspule auf, an die ein hochfrequentes Signal anzulegen ist. Der eine Anschluß der Hochfrequenzspule ist mit dem Sensor der andere Anschluß mit einer Kapazität verbunden. Weiterhin weist das erfindungsgemäße Meßsystem ein Potentiometer und einen Gleichrichter auf. Das Potentiometer, das einen einstellbaren Abgriff aufweist, ist parallel zur Hochfrequenzspule geschaltet. Der Eingang des Gleichrichters ist mit dem Abgriff des Potentiometers verbunden. Der Ausgang gibt ein Ausgangssignal des Meßsystems aus. Das Abgleichen erfolgt mittels des Abgriffs des Potentiometers.

Ein erfindungsgemäßes Computerprogramm weist Programmcodemittel auf, um alle Schritte des vorstehend beschriebenen Verfahrens durchzuführen. Das Computerprogramm wird auf einer elektronischen Recheneinheit zur Ausführung gebracht. Als elektronische Recheneinheit kann dabei bspw. der Mikroprozessor dienen, der als Abgleicher vorgesehen ist.

Ein erfindungsgemäßes Computerprogrammprodukt weist Programmcodemittel auf, die auf einem computerlesbaren Datenträger gespeichert sind, um das erfindungsgemäße Verfahren durchzuführen. Die Programmcodemittel sind auf einem computerlesbaren Datenträger gespeichert. Als geeignete Datenträger können EE-PROMs, Flashmemories, aber auch CD-ROMs, Disketten oder Festplattenlaufwerke verwendet werden.

Weitere Vorteile und Ausgestaltungen der Erfindung ergeben sich aus der Beschreibung und der beiliegenden Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand von Ausführungsbeispielen in der Zeichnung dargestellt und wird im folgenden unter Bezugnahme auf die Zeichnung näher erläutert.
- Figur 1: zeigt in schematischer Darstellung eine bevorzugte Ausführungsform einer erfindungsgemäßen Vorrichtung.
- Figur 2: zeigt eine bevorzugte Ausführungsform eines erfindungsgemäßen Meßsystems im Blockschaltbild.

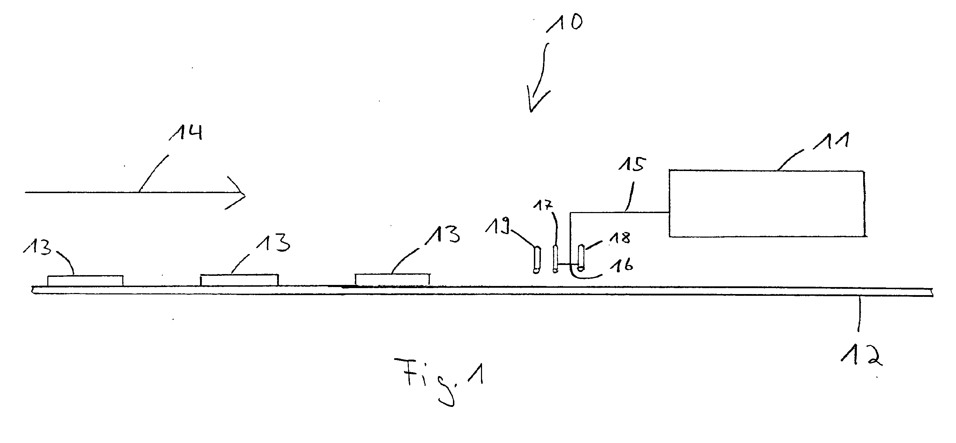

Figur 1 zeigt eine erfindungsgemäße Vorrichtung 10 zum Messen der Feuchte in einer Anzahl von Werkstücken. Die Vorrichtung 10 weist ein Meßsystem 11 und ein Förderband 12 auf. Auf dem Förderband 12 befinden sich drei Werkstücke 13, in diesem Fall Holzbretter, die mittels des Förderbands 12 in Richtung eines Pfeils 14 transportiert werden.

Das Meßsystem 11 ist über eine Verbindungsleitung 15 mit einem Sensor 16 verbunden. Der Sensor 16 ist in diesem Fall eine Elektrode einer Kapazität. An dem Sensor 16 ist eine erste Lichtschranke 17 und eine zweite Lichtschranke 18 angeordnet. In einigem Abstand von der ersten Lichtschranke 17 entgegen der Bewegungsrichtung der Werkstücke 13 ist eine dritte Lichtschranke 19 angeordnet.

Im Betrieb werden die Werkstücke 13 von dem Förderband 12 in Richtung des Pfeils 14 bewegt. Sobald das Werkstück 13 von der dritten Lichtschranke 19 erfaßt wird, erfolgt der Abgleich des Systems. Mit diesem Abgleich werden geänderte Rahmenbedingungen kompensiert. Das Werkstück 13 gelangt anschließend, von dem Förderband 12 transportiert, in den Bereich des Sensors 16. Hierbei wird das Werkstück 13 zunächst von der ersten Lichtschranke 17 erfaßt. Damit beginnt der Meßzyklus, in dem die Feuchte des Werkstücks 13 vorzugsweise mehrmals bestimmt wird. Sobald das Werkstück 13 sich nicht mehr unterhalb des Sensors 16 befindet, wird dies von der zweiten Lichtschranke 18 registriert und dem Meßsystem 11 angezeigt. Der Meßzyklus ist damit beendet. Sobald das nächste Werkstück 13 von der dritten Lichtschranke 19 erfaßt wird, erfolgt ein erneuter Abgleich des Meßsystems 11. Haben sich in der Zeit zwischen zwei Meßzyklen äußere Rahmenbedingungen, wie bspw. die Luftfeuchtigkeit und/oder die Umgebungstemperatur, geändert, wird dies durch den Abgleich des Meßsystems 11 kompensiert.

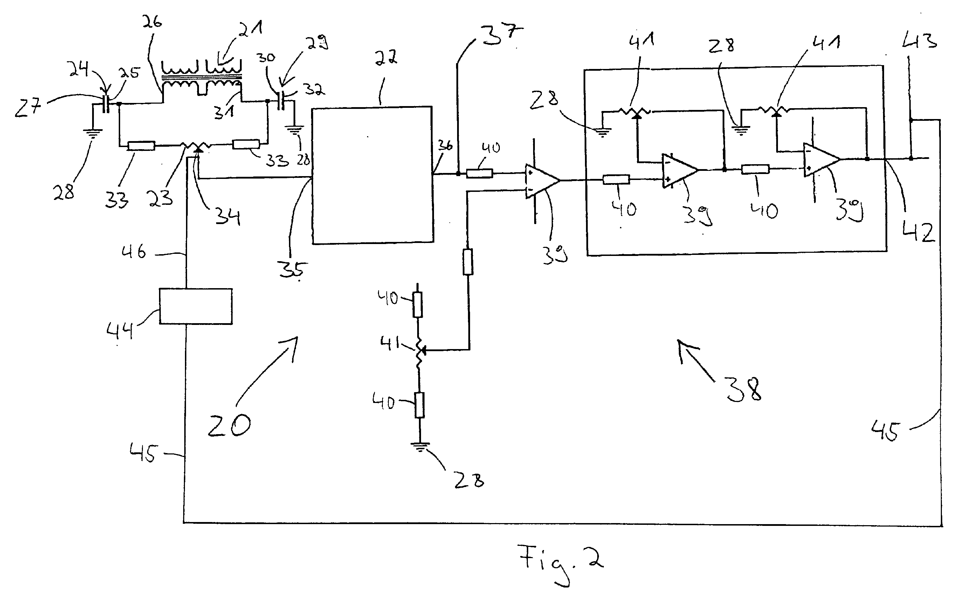

Figur 2 zeigt ein bevorzugtes Meßsystem 20 im Blockschaltbild. Dieses Meßsystem 20 umfaßt eine Hochfrequenzspule 21, einen Gleichrichter 22, ein Potentiometer 23 und eine erste Kapazität 24.

Eine erste Elektrode 25 der ersten Kapazität 24 ist mit einem ersten Anschluß 26 der Hochfrequenzspule 21 verbunden. Eine zweite Elektrode 27 der ersten Kapazität 24 ist mit Masse 28 verbunden.

Symmetrisch zur ersten Kapazität 24 ist eine zweite Kapazität 29 vorgesehen, deren eine Elektrode 30 mit einem zweiten Anschluß 31 der Hochfrequenzspule 21 und deren andere Elektrode 32 mit Masse 28 verbunden ist.

Die eine Elektrode 30 der zweiten Kapazität 29 wird durch einen Sensor 30 gebildet. Während einer Messung befindet sich das Werkstück 13 zwischen der einen Elektrode 30 bzw. dem Sensor 30 und der anderen Elektrode 32, die mit Masse 28 verbunden ist. Der Wert der Kapazität 29 ist somit abhängig von der Beschaffenheit des Werkstücks 13 und somit auch von dessen Feuchte.

Das Potentiometer 23 ist parallel zu der Hochfrequenzspule 21 geschaltet. In Reihe zum Potentiometer 23 sind außerdem zwei Widerstände 33 geschaltet. Das Potentiometer 23 weist einen Abgriff 34 auf, an dem sich bei Anlegen eines hochfrequenten Eingangssignals an der Hochfrequenzspule 21 eine elektrische Wechselgröße einstellt.

Der Abgriff 34 ist mit einem Eingang 35 des Gleichrichters 22 verbunden. Die Wechselgröße am Abgriff wird im Gleichrichter 22 in eine Gleichgröße gewandelt und diese Gleichgröße wird an einem Ausgang 36 des Gleichrichters 22 als Ausgangsgröße 37 des Meßsystems 20 ausgegeben.

Diese Ausgangsgröße 37 ist abhängig vom Wert der Kapazität 29, also von der Feuchte des Werkstücks 13.

Zu Beginn der Messung wird bei einer Leermessung der Abgriff 34 des Potentiometers 23 so eingestellt, daß sich ein möglichst geringer Wert, der Referenzwert, als Ausgangsgröße 37 einstellt. Je geringer dieser Wert ist, desto größer ist der Dynamikbereich bei den nachfolgenden Messungen. Nach jedem Meßzyklus wird das Potentiometer 23 so eingestellt, daß dieser Wert wieder anliegt. So können die Auswirkungen geänderter Rahmenbedingungen kompensiert werden.

Während des Meßvorgangs befindet sich das Werkstück 13 zwischen den Elektroden 30, 32 der zweiten Kapazität 29, also zwischen dem Sensor 30 und Masse 28. Der Wert der zweiten Kapazität 29 ist abhängig von der Feuchte des Werkstücks 13. Die sich einstellende Größe am Abgriff 34 ist wiederum abhängig von dem Wert der zweiten Kapazität 29. Folglich ergibt sich ein Zusammenhang zwischen der Ausgangsgröße 37 und der Feuchte des Werkstücks 13.

Weiterhin ist ein Verstärker 38 zum Verstärken der Ausgangsgröße 37 vorgesehen. Dieser Verstärker 38 umfaßt drei Operationsverstärker 39 und eine Reihe von Widerständen 40. Mittels dreier weiterer Potentiometer 41 ist der Verstärker 38 abgleichbar. Am Ausgang 42 des Verstärkers 38 liegt das verstärkte Ausgangssignal 37, in diesem Fall die Meßgröße 43, an, die die Feuchte des gemessenen Werkstücks 13 repräsentiert.

Als Abgleicher des Meßsystems 20 ist bei der gezeigten Ausführungsform ein Mikroprozessor 44 vorgesehen. Dieser empfängt über eine Datenleitung 45 die Meßgrößen 43, zeichnet diese auf und verarbeitet diese. Eine Mittelwertbildung findet beispielsweise auch mit Hilfe des Mikroprozessors 44 statt. Der Mikroprozessor 44 ist zweckmäßigerweise mit geeigneten Darstellungsmitteln verbunden, um die Meßergebnisse darstellen zu können.

Der Mikroprozessor 44 ist über eine Steuerleitung 46 mit dem Potentiometer 23 bzw. dessen Abgriff 34 verbunden. Beim Abgleichen empfängt der Mikroprozessor 44 die Meßgröße 43 der Leermessung über die Datenleitung 45. Da der Mikroprozessor 44 den einzustellenden Referenzwert kennt, kann er über die Steuerleitung 46 den Abgriff 34 so einstellen, daß sich als Meßgröße 43 der Leermessung der Referenzwert ergibt.

## Patentansprüche

1. Verfahren zum Messen der Feuchte in einer Anzahl von Werkstücken (13), bei dem die Werkstücke (13) nacheinander an einem Sensor (16, 30) vorbeigeführt werden und in jeweils einem Meßzyklus die Feuchte in einem der Werkstücke (13) mindestens einmal gemessen wird, während das betreffende Werkstück (13) an dem Sensor (16, 30) vorbeigeführt wird, wobei die Feuchte mittels eines mit dem Sensor verbundenen Meßsystems (11, 20) ermittelt und nach jedem Meßzyklus ein Abgleich des Meßsystems (11, 20) durchgeführt wird.

2. Verfahren nach Anspruch 1, bei dem die Feuchte mittels eines kapazitiven Meßverfahrens ermittelt wird.

3. Verfahren nach Anspruch 1 oder 2, bei dem die Messung kontaktlos erfolgt.

4. Verfahren nach einem der Ansprüche 1 bis 3, bei dem eine Mittelwertbildung durchgeführt wird.

5. Verfahren nach einem der Ansprüche 1 bis 4, bei dem die Werkstücke (13) mit einer Geschwindigkeit von 400 m/min an dem Sensor (16, 30) vorbeigeführt werden.

6. Verfahren nach einem der Ansprüche 1 bis 5, bei dem die Feuchte jedes Werkstücks (13) in 1/10 mm-Abständen bestimmt wird.

7. Vorrichtung zum Messen der Feuchte in einer Anzahl von Werkstücken (13), insbesondere zur Durchführung eines Verfahrens nach einem der Ansprüche 1 bis 6, mit
- einem Sensor (16, 30),
- einem mit dem Sensor (16, 30) verbundenen Meßsystem (11, 20),
- einem Fördermittel zum Vorbeiführen der Werkstücke (13) an dem Sensor (16, 30),
wobei ein Abgleicher zum Abgleichen des Meßsystems (11, 20) vorgesehen ist.

8. Vorrichtung nach Anspruch 7, bei der als Abgleicher ein Mikroprozessor (44) dient.

9. Vorrichtung nach Anspruch 7 oder 8, bei der das Meßsystem (11, 20) folgendes aufweist:
- eine Hochfrequenzspule (21), an die ein hochfrequentes Eingangssignal anzulegen ist, mit deren einem Anschluß (26) eine erste Kapazität (24) und mit deren anderem Anschluß (31) der Sensor (16, 30) verbunden ist,
- ein Potentiometer (23), das parallel zur Hochfrequenzspule (21) geschaltet ist und einen einstellbaren Abgriff (34) aufweist, und
- einen Gleichrichter (22), dessen Eingang (35) mit dem Abgriff (34) des Potentiometers (23) verbunden ist und dessen Ausgang ein Ausgangssignal (37) des Meßsystems (11, 20) ausgibt.

10. Vorrichtung nach Anspruch 9, bei der das hochfrequente Eingangssignal eine Frequenz von 50 MHz aufweist.

11. Vorrichtung nach einem der Ansprüche 7 bis 10, bei der zwei Lichtschranken (16, 17) zum Starten und Beenden eines Meßzyklusses vorgesehen sind.

12. Vorrichtung nach einem der Ansprüche 7 bis 11, bei der eine dritte Lichtschranke (19) zum Starten des Abgleichens vorgesehen ist.

13. Meßsystem mit
- einer Hochfrequenzspule (21), an die ein hochfrequentes Eingangssignal anzulegen ist, mit deren einem Anschluß (26) eine erste Kapazität (24) und mit deren anderem Anschluß (31) der Sensor (16, 30) verbunden ist,
- einem Potentiometer (23), das parallel zur Hochfrequenzspule (21) geschaltet ist und einen einstellbaren Abgriff (34) aufweist, und
- einem Gleichrichter (22), dessen Eingang (35) mit dem Abgriff (34) des Potentiometers (23) verbunden ist und dessen Ausgang (36) ein Ausgangssignal (37) des Meßsystems (11, 20) ausgibt.

14. Meßsystem nach Anspruch 13, bei dem ein Verstärker (38) zum Verstärken des Ausgangssignals (37) vorgesehen ist.

15. Meßsystem nach Anspruch 14, bei dem der Verstärker (38) abgleichbar ist.

16. Computerprogramm mit Programmcodemitteln, um alle Schritte eines Verfahrens nach einem der Ansprüche 1 bis 6 durchzuführen, wenn das Computerprogramm auf einem Computer oder einer entsprechenden Recheneinheit ausgeführt wird.

17. Computerprogrammprodukt mit Programmcodemittteln, die auf einem computerlesbaren Datenträger gespeichert sind, um ein Verfahren nach einem der Ansprüche 1 bis 6 durchzuführen, wenn das Computerprogramm auf einem Computer oder einer entsprechenden Recheneinheit ausgeführt wird.
